# EUROPEAN PATENT APPLICATION

(11) **EP 1 650 561 A1**
(43) Date of publication of application: **26.04.2006**
(21) Application number: 05466014.7
(22) Date of filing: 11.10.2005
(51) Int. Cl.: G01N 33/36, B61B 7/04, B65H 61/00

(54) **Method of measuring of yarn extention and a device for carrying out the method**

(30) Priority: 22.10.2004 CZ 20041058
(71) Applicant: Vyzkumny Ustav Textilnich Stroju Liberec a.s., 461 19 Liberec (CZ)
(72) Inventor: Sidlof, Pavel, 460 14 Liberec (CZ); Skop, Petr, 460 01 Liberec (CZ); Kloucek, Pavel, Liberec (CZ); Cejka, Vaclav, 460 13 Liberec (CZ); Busek, Martin, 460 06 Liberec (CZ)
(74) Representative: Musil, Dobroslav

(57) **Abstract**

The invention relates to the measuring method of yarn (1) extension and of similar longitudinal formation during which the continuous stream of the yarn (1) is continuously loaded with a tensile force of a defined value and the values, from which the continuous yarn (1) extension is determined, are being continuously measured in the measuring system.

The invention also related to the device for measuring of yarn (1) extension and of similar longitudinal formations which contains the continuous yarn (1) feeding mechanism into the measuring zone (17) and the continuous yarn(1) delivery mechanism from the measuring zone (17) when in the measuring zone (17) there is positioned at least one sensor detecting the values for determination of continuous yarn (1) extension coupled with the recording and/or evaluation device and the equipment also contains the control module (14) at least for control of feeding and delivery of yarn (1) to and from the measuring zone (17).

## Description

### Technical field

The invention relates to the method of measuring of yarn extension and of similar longitudinal formations.

The invention relates also to the device for measuring of yarn extension and of similar longitudinal formations.

### Background art

The testing procedures and evaluation of mechanical properties of yarns, threads and of similar longitudinal formations are known, like measurement of strength and elongation of threads and yarns at breakage and also measurement of longitudinal mass irregularity.

Measuring of strength and elongation at breakage is performed on breaking machines at which the standardised length of yarn is manually fastened into the clamping device and the values of extension of yarn or of thread upon stress are measured. Strength and extension of yarn is usually stated as a value at break, which is applied especially for estimate of number of stoppage of manufacturing machines due to breakage of processed yarns or threads. It is possible to make the estimate much more precise, but only by performing a large number of tests (up to several thousands per one hour). For this purpose e.g. Tensojet, the breaking device, is being used at which the yarn is blown by air in between two pairs of rotating rollers, whereas the distance of both pairs corresponds to the standardised tested length of yarn. Both pairs of rollers are rotating synchronously. Always one roller from each pair of rollers is fully circular while on the second roller from the pair, a portion of a circular arch is cut off and the yarn is blown into this cut-off area when the roller is turned with this cut-off area into the position parallel with longitudinal axis of the channel. As the rollers rotate the yarn is clamped between the pairs of rollers and thanks to different peripheral speed between the pair of rollers, the yarn is stretched until it breaks. At this moment the tensile force of yarn stress is measured, this using the force sensor positioned approximately in the middle of distance between the rollers where the channel with the yarn is bent slightly. The value of yarn extension is determined from the dimension of mutual angular rotation of the pair of rollers at yarn stretching.

Upon measuring the longitudinal mass irregularity e.g. using the UsterTester equipment, the measured yarn moves between plates of measuring condenser, which is connected into the tuned alternating circuits. According to variation of mass content of the yarn between the condenser plates, the output signal changes from which the longitudinal mass irregularity of yarn or thread is determined. The output signal of greater yarn lengths is being analysed and various parameters of yarn or thread are calculated based on these data. For example the courses of deviations beginning from the medium value are being determined from which the spectrograms with spot or column image are being composed that serve for detection of periodical irregularities of yarn or thread that cause the defects of appearance in the final product (fabric, knitted fabric). Further to this, the medium quadratic mass irregularity CV (%) is being determined that expresses the swing of mass irregularity around the medium value.

The main disadvantage of existing state of the art is that these are discontinuous methods of extension measurement, i.e. that only a portion of the yarn out of the total length is measured and the rest of yarn is lost due to manipulation without being measured. The measurement results of yarn strength and extension are based on the statistical evaluation of a great number of measurements. Through this a number of important information is lost, especially about the longitudinal variation of strength and extension in the whole measured section of yarns. Another disadvantage of existing state of the art of measurement of strength and extension of yarns and threads and similar longitudinal formations is that the values are being detected e.g. at breakage, which is highly above the values of real stress of yarns and threads upon their processing into textile fabrics and also above the real values of stress of textile fabrics during their usage. Further disadvantage of the existing state of the art is that it is not possible to perform advantageous analyses also with possible searching for conjunction between the mass irregularity, extension and strength of yarn or threads and of similar longitudinal formations.

The goal of the invention is to eliminate or at least to minimise the disadvantages of present state of the art.

### The principle of invention

The goal of the invention has been reached by a measurement method of extension of yarns and of similar longitudinal formations, whose principal consist in that the continual flow of yarn is continuously stressed with tensile force of a defined value and the values in the measuring system from which the continuous extension of yarn is determined are being measured continuously.

The advantage of this method is that the value of tensile force acting upon measurement to the yarn can be set to the level of the expected average or even peak load of yarn at the following processing of yarn, e.g. at weaving or knitting, and simultaneously to measure the momentary extension of yarn. The variation of values of yarn extension can be then analysed statistically, at the same time it is possible to determine e.g. the mean values, the conclusive deviations, the limit values, swing, etc. this always for the whole measured length of yarn or only for specified shorter sections. At the same time it is possible to identify influence of individual wave lengths to the results of measurement, this through stating the length variation curve. From the course of variation of yarn extension around the medium value it is possible, through methods of spectral analysis, to receive the amplitudes of periodical changes in yarn extension. At the expressive spectrum it is possible to find connection between the wavelengths and functioning of individual working elements of manufacturing machines. It is also possible to monitor and describe connections between the changes measured in yarn extension and the longitudinal mass irregularity of yarn, possibly the longitudinal diameter irregularity of yarn.

Preferred embodiments of this method are contained in patent claims 2 to 5.

The principle of device for measuring of the yarn extension and of similar longitudinal formations consist in a fact that the device contains the continuous yarn feeding mechanism into the measuring zone and continuous yarn delivery mechanism from the measuring zone when in the measuring zone there is at least one sensor detecting the values for determining the continuous yarn extension; this sensor is coupled with recording and/or evaluating equipment and this equipment also contains the controlling module at least for control of feeding and delivery of yarn into and from the measuring zone.

Such arrangement is relatively simple and is able to give a quality and exact results of measurement.

Preferred embodiments of this device are contained in the patent claims 7 to 20.

### Description of the drawing

The invention is schematically shown in the drawing.

### Examples of embodiment

The method of measuring of yarn **1** extension and of similar longitudinal formations will be described on the principle of functioning of device for measuring of yarn extension measurement and of similar longitudinal formations.

The method of measuring of yarn **1** extension and of similar longitudinal formations consists in the fact that the measured yarn passes continuously through the measuring equipment and is continuously loaded to the in advance determined constant value of tensile force whereas the yarn **1** extension is continuously measured at the same time. The value of tensile force can be set e.g. to the level which corresponds to the expected average or even peak values of yarn **1** stress upon the subsequent processing of the yarn **1,** e.g. at weaving or knitting and to measure the instantaneous yarn **1** extension at the same time. Variation of yarn **1** extension values is being statistically analysed when e.g. the mean value, conclusive variation, limit values, swing etc. are being determined, which can be determined either for a whole length of the yarn or only for shorter sections. At the same time it is possible to identify influence of individual wave lengths to the results of measurement, this through determining the length variation curve. From the course of variation of yarn **1** extension around the mean value it is possible, through methods of spectral analysis, to receive the amplitudes of periodical changes in yarn **1** extension. At the expressive spectrum it is possible to find connection between the wave lengths and functioning of individual working elements of that respective manufacturing machine or machines. It is also possible to watch and describe connections between the changes measured in yarn 1 extension and the longitudinal mass irregularity of yarn **1**, possibly the longitudinal diameter irregularity of yarn **1.**

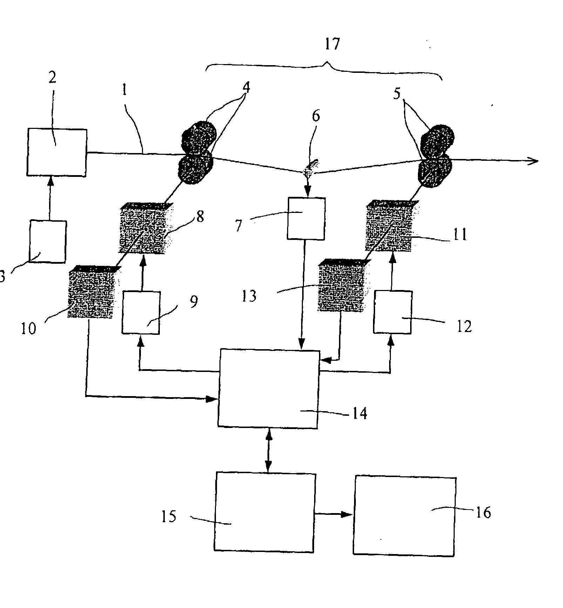

The figure 1 shows an example of embodiment of device for continuous measurement of yarn **1** extension at the selected tensile force of yarn **1**. To ensure a relatively small and constant tensile force in yarn **1** before the measuring zone **17** there is the rewinder **2** included between the yarn bobbin **3** and the feeding rollers **4** .

From rewinder **2** the yarn **1** is drawn off with a pair of feeding rollers **4** into the measuring zone **17**. The feeding rollers **4** are connected to the drive **8** or to the servo-drive controlled with a frequency changer **9** which is coupled with the controlling module **14**. The speed of feeding the yarn **1** into the measuring zone **17** is measured with the sensor **10** which is coupled with feeding rollers **4**. It is advantageous if the sensor **10** is created by an incremental sensor.

Further to this the device contains the draw-off rollers **5** for drawing off the yarn **1** from the measuring zone **17**. Rotation of the draw-off rollers **5**, namely of their rotational speed is controlled through the drive **11** or the servo-drive controlled by the frequency changer **12** according to the control data from the control module **14**. Rotation of the draw-off rollers **5** is monitored by a sensor **13** coupled with the draw-off rollers **5**. It is advantageous if the sensor **13** is created by an incremental sensor.

In the shown example of embodiment the control module **14** contains the means (including the respective algorithms) for regulation of tensile force in yarn **1** in the measuring zone **17** according to given values and also includes means (including respective algorithms) for evaluation of yarn **1** extension in the measuring zone **17**. The control module **14** next to this, in the shown example of embodiment, contains means for measuring the yarn **1** length fed into the measuring zone **17.**

For input of parameters at which the measuring of continuous extension is performed, like the speed of yarn **1** movement, value of tensile force in yarn **1** and the measured yarn **1** length, the control module **14** is provided with suitable means like the keyboard etc., or the control module **14** is provided with means for connection, with an appropriate cable or a suitable technology of wireless data transfer, control computer **15.**

The control module **14** may, at the same time, serve as the measuring module (as shown in the figure) which ensures the data collection from measuring, possibly the evaluation of measuring and also the displaying of measured results, calculations, analyses and possibly the archiving of data.

The measuring itself, depending on the fact whether the control computer 15 is used or is not used, may be set, started and cancelled either using only the means of control module **14** or this can be done by means of the control computer **15.**

As the need may be, the control module **14** is equipped with means for data collection from measuring and for measuring evaluation and possibly with means for displaying the measured results, calculations, analyses and means for data archiving.

The control computer **15** with advantage contains at least means for evaluation of measurement, for displaying the measured results, calculations, analyses and also the means for archiving the data.

Next to this, the control module **14** may also be equipped with means for connecting the printer **16** for direct print of measurement results or other outputs of measurement. At the execution of equipment with control computer **15 ,** the printer **16** may also be connected to the control computer **15**.

In another not represented example of embodiment, the control module 14 is fully superseded with a control computer **15** of the PC type provided with provisions (software or special hardware) for control of measuring and processing of measured results.

In the shown example of embodiment, a suitable sensor **of 6** tensile force in the yarn **1** is assigned in the measuring zone **17** of yarn **1 .** The signal from the **sensor 6** of tensile force in the **yarn 1** is transferred into the measuring module i.e. in the shown example of embodiment into the control module **14,** which is at the same time also the measuring module, possibly the signal of the sensor **6** of the tensile force in the yarn **1** before entering the measuring module or the control module **14,** is being amplified in the amplifier **7**. The yarn **1** length in the measuring zone is, at the not represented example of embodiment, adjustable, i.e. the distance between the feeding rollers **4** and draw-off rollers **5** can be adjusted. In the not represented example of embodiment, the sensor **6** in the measuring zone **17** is not used, but the tensile force in the yarn **1** is being determined from the torque moment of the drive **11** of the draw-off rollers **5** and/or of a drive **8** of feeding rollers **4.**

The yarn **1** extension is continuously being determined from the value of instantaneous rotation speed of feeding rollers **4,** from value of tensile force in the yarn **1** measured by the sensor **6** of the tensile force in the yarn **1** and from the value of instantaneous rotation speed of draw-off rollers **5.** From the data measured with corresponding sensors it is possible to determine also the yarn **1** extension in the smaller length sections of yarn **1** than the yarn **1** length in the measuring zone **17**, i.e. between the feeding rollers **4** and draw-off rollers **5**. The instantaneous 1 yarn extension may also be determined from the difference of angle of rotation or the difference of rotation speed of feed rollers **4** and of draw-off rollers **5.**

The feed rollers **4** and the draw-off rollers **5** are, in the not represented example of embodiment, provided with provisions against yarn **1** slippage and/or against winding the yarn **1** onto the feeding and draw-off rollers **4, 5.** As an example, of embodiment the rollers **4, 5** are as a combination of metal and rubber roller, etc.

To make the measurement precise, possibly for creation of an equipment for a complex detection of yarn properties, it is possible to integrate at least some additional sensing device into the equipment like the device for measuring of yarn **1** diameter on input, possibly on output of the measuring zone **17** which may contain e.g. the CCD sensing element of yarn **1**. Next to this, it is possible to integrate the device for measuring of mass irregularity of the yarn **1** before the input of the measuring zone **17** e.g. in the form of capacity sensor for measuring of mass deviations of the yarn **1** on the input into the measuring zone **17**. If we include further data into the continual measuring of yarn **1** extension this measuring can become much more precise or can become a complex measuring of yarn **1** properties .

### Industrial applicability

The invention is applicable first of all in the textile production, but generally it can be utilised for measuring of longitudinal formations similar to yarns.

### List of referential markings

1 yarn
2 rewinder
3 bobbin
4 feeding rollers
5 draw-off rollers
6 sensor of draw-off force in the yarn
7 amplifier
8 drive
9 frequency changer
10 sensor
11 drive
12 frequency changer
13 sensor
14 control module
15 control computer
16 printer
17 measuring zone

## Claims

1. A method of measuring of yarn extension and of similar longitudinal formations, **characterised by** that the continuous stream of yarn (1) is being continuously loaded with a tensile force of a defined value and the values are measured, in the measuring system, from which the continuous yarn (1) extension is determined.

2. A method as claimed in the claim 1, **characterised by** that the yarn (1) is loaded with a tensile force of a defined value in the measuring zone (17), in which the values are measured, out of which the continuous yarn (1) extension is being determined

3. A method as claimed in any of the claims 1 or 2, **characterised by** that the yarn (1) is loaded with a tensile force whose value corresponds to the yarn (1) load during the further processing.

4. A method as claimed in any of the claims 1 to 3, **characterised by** that on the yarn (1) at least one additional measurement of other yarn (1) parameters is being carried out.

5. A method as claimed in claim 4, **characterised by** that the additional measuring is the measuring of yarn (1) cross section and/or measuring of mass irregularity of the yarn (1).

6. A device for measuring of yarn extension and of similar longitudinal formations, **characterised by** that it contains the mechanism for continuous feeding of the yarn (1) into the measuring zone (17) and for the continuous yarn (1) delivery from the measuring zone (17), when in the measuring zone (17) there is at least one sensor of values for determination of the continuous yarn (1) extension which is coupled with recording and/or evaluation equipment and the equipment further contains the control module (14) at least for controlling of feeding and delivery of yarn (1) into and from measuring zone (17).

7. A device as claimed in claim 6, **characterised by** that it contains the yarn rewinder (2) behind which are included the feeding rollers (4) of the yarn (1) into the measuring zone (17), and behind which are included the draw-off rollers (5) of the yarn (1) from the measuring zone (17).

8. A device as claimed in claim 7, **characterised by** that the feeding and draw-off rollers (4, 5) are coupled with a drive (8, 11) which is controlled by means of a frequency changer (9, 12) coupled with the control module (14).

9. A device as claimed in claim 8 **characterised by** that in the area of feeding and draw-off rollers (4, 5) there is positioned at least one sensor (10, 13) sensing the speed of yarn (1) coupled with the control module (14).

10. A device as claimed in claim 9, **characterised, by** that the sensor (10, 13) is created by a sensor sensing the rotation of feeding and draw-off rollers (4, 5).

11. A device as claimed in any of claims 6 to 10, **characterised by** that the control module (14) contains the means for data collection from the device and/or for evaluation and/or for analysis of data from device for measuring and/or is provided with means for archiving and/or transfer and/or displaying and/or printing of data and/or results of measuring and/or is provided with means for connection of these means.

12. A device as claimed in any of claims 6 to 11, **characterised by** that the control module (14) is provided with means for input of measuring parameters and/or is provided with the means for connection of means for input of measuring parameters and/or is provided with means for connection of control computer (15).

13. A device as claimed in any of claims 6 to 11, **characterised by** that the control module (14) is directly created by the computer (15) of PC type which has means for control of measurement, collection, storing and export of data from measurement and for evaluation, analysis and visualisation of results from measurement.

14. A device as claimed in any of claims 6 to 13, is **characterised by** that the sensor of values for determination of continuous yarn (1) extension is created of the sensor (6) of tensile force in the yarn (1) included in the measuring zone (17) and coupled with the measuring module (14).

15. A device as claimed in claim 14, **characterised by** that the amplifier (7) is included between the sensor (6) of the tensile force in the yarn (1) and the measuring module (14).

16. A device as claimed in any of claims 6 to 15, **characterised by** that the measuring zone (17) has an adjustable length.

17. A device as claimed in any of claims 6 to 13, 15, 16, **characterised by** that the sensor of values for determination of continuous yarn (1) extension is created by the sensor sensing the torque of the drive (11) of draw-off rollers (5) and/or of the drive of (8) feeding rollers (4).

18. A device as claimed in any of claims 7 to 17, is **characterised by** that the feeding rollers (4) and the draw-off rollers (5) are equipped with means against slippage of yarn (1) and/or against yarn (1) winding onto the rollers (4, 5).

19. A device as claimed in any of claims 6 to 18, **characterised by** that it also contains at least one additional sensing device.

20. A device as claimed in claim 19, **characterised by** that the additional sensing device is from the group of device for measuring of yarn (1) cross-section and/or device for measuring of mass irregularity of yarn (1).
